# FASCICULE DE BREVET EUROPEEN

(11) **EP 0 739 620 B1**
(45) Date de publication et mention de la délivrance du brevet: **08.11.2000**
(21) Numéro de dépôt: 95400923.9
(22) Date de dépôt: 25.04.1995
(51) Int. Cl.: A61K 7/06

(54) **Compositions pour le traitement et la protection des cheveux, à base de céramide et de polymères à groupements cationiques**
Haarpflege- und Haarschutzmittel auf Basis von Ceramid und Polymeren mit kationischen Gruppen
Compositions for hair treatment and hair protection, based on a ceramide and a cation containing polymer

(43) Date de publication de la demande: 30.10.1996
(73) Titulaire: L'OREAL, 75008 Paris (FR)
(72) Inventeur: Dubief, Claude, F-78150 Le Chesnay (FR); Cauwet, Danièle, F-75011 Paris (FR)
(74) Mandataire: Casalonga, Axel

(56) Documents cités:
- EP-A- 0 278 505
- EP-A- 0 474 023
- EP-A- 0 495 624
- WO-A-92/06982
- WO-A-94/03151
- FR-A- 2 679 770

## Description

La présente invention est relative à des compositions destinées au traitement et à la protection des cheveux et contenant dans un milieu cosmétiquement acceptable, au moins un céramide et/ou glycocéramide et un polymère cationique contenant des atomes d'azote dans la chaîne principale.

L'invention est également relative au procédé de traitement cosmétique mettant en oeuvre de telles compositions.

On connaît déjà dans l'état de la technique des formulations capillaires permettant de traiter les cheveux abîmés par les intempéries ou des traitements capillaires non appropriés. On a déjà utilisé dans ce but des polymères cationiques qui ont pour avantage d'améliorer ces propriétés cosmétiques, entre autre le démêlage et la douceur des cheveux humides et séchés et également de protéger les fibres capillaires de ces agressions.

Cependant, certains polymères cationiques ont des propriétés de démêlage non satisfaisantes et ont tendance par exemple, lors de superpositions de traitements, à alourdir la chevelure et à la rendre rèche.

On connaît également les céramides ou les glycocéramides qui ont déjà été associés à des esters de cholestérol dans le but de protéger la fibre capillaire.

FR-A-2 679 770 révèle des dispersions cationiques pour le traitement des cheveux ou de la peau contenant au moins un céramide ou glycocéramide et au moins un agent tensio-actif cationique de structure d'un sel d'ammonium quaternaire.

L'application sur cheveux de ces dernières compositions ou des céramides seuls conduit cependant à des performances cosmétiques insuffisantes, tant sur cheveux mouillés que sur cheveux séchés.

Les inventeurs ont découvert que, de façon surprenante, l'association de céramides ou de glycocéramides avec certains polymères cationiques, conduisait à des propriétés cosmétiques particulièrement intéressantes, notamment en ce qui concerne le démêlage humide.

Ils ont constaté en particulier que l'association avait un effet de synergie qui n'est pas simplement l'addition des propriétés des deux composants, notamment dans des milieux non détergents ayant une concentration faible ou nulle en agents tensio-actifs lavants.

La demanderesse a constaté que cet effet était plus particulièrement obtenu en associant avec les céramides ou glycocéramides des polymères cationiques comportant des groupements amine primaires, secondaires, tertiaires ou ammonium quaternaire dans la chaîne principale et ayant une viscosité à 1% en poids dans l'eau inférieure à 15 m Pa.s.

On a envisagé dans la demande WO 93/02656 d'utiliser des dispersions cationiques de céramides ou glycocéramides, les agents tensio-actifs cationiques utilisés dans de telles dispersions sont cependant différents des polymères cationiques mis en oeuvre conformément à l'invention, et ne permettent pas d'obtenir l'effet recherché, entre autre ils ne permettent pas de protéger les fibres.

Un objet de l'invention est donc constitué par une composition non lavante destinée au traitement et à la protection des cheveux à base de céramide et/ou de glycocéramide et de polymères cationiques.

L'invention a également pour objet un procédé de traitement cosmétique des cheveux mettant en oeuvre une telle composition.

D'autres objets de l'invention apparaîtront à la lecture de la description et des exemples qui suivent.

La composition destinée au traitement et à la protection des cheveux, conforme à l'invention, est essentiellement caractérisée par le fait qu'elle contient dans un milieu cosmétiquement acceptable au moins un céramide et/ou glycocéramide et au moins un polymère cationique comportant des atomes d'azote dans la chaîne principale et dont la viscosité à 1% dans l'eau est inférieure ou égale à 15 m Pa.s, la composition contenant moins de 4% d'agents tensio-actifs anioniques et/ou amphotères et/ou zwittérioniques.

Les polymères cationiques utilisables conformément à l'invention sont choisis parmi les polymères cationiques comportant des groupements amine primaire, secondaire, tertiaire ou quaternaire dans la chaîne polymérique principale. Ils ont un poids moléculaire supérieur à 500, de préférence 1.000. Ces polymères présentant par ailleurs les propriétés de viscosité définies ci-dessus.

Les polymères cationiques particulièrement utilisables conformément à l'invention, sont choisis parmi :
(1) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont décrits dans les brevets français 2.162.025 et 2.280.361.
(2) Les polyaminoamides solubles dans l'eau préparés en particulier par polycondensation d'un composé acide avec une polyamine. Ces polyaminoamides peuvent être réticulés par une épihalohydrine, un diépoxyde, un dianhydride, un anhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé; l'agent réticulant étant utilisé dans des proportions allant de 0,025 à 0,35 mole par groupement amine du polyaminoamide.
   Ces polyaminopolyamides peuvent être alcoylés ou s'ils comportent une ou plusieurs fonctions amines tertiaires, quaternisés. De tels polymères sont décrits en particulier dans les brevets français 2.252.840 et 2.368.508.
(3) Les dérivés de polyaminoamides résultant de la condensation de polyalcoylène polyamines avec des acides polycarboxyliques suivie d'une alcoylation par des agents bifonctionnels. On peut citer par exemple les polymères acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont décrits dans le brevet français 1.583.363.
   Parmi ces dérivés, on peut citer plus particulièrement les polymères acide adipique/diméthylaminohydroxypropyl/diéthylène triamine vendus sous la dénomination "CARTARETINE F, F₄ ou F₈" par la Société SANDOZ.
(4) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre la polyalkylène polyamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont décrits en particulier dans les brevets américains 3.227.615 et 2.961.347.
   Des polymères de ce type sont en particulier commercialisés sous la dénomination "HERCOSETT 57" par la Société HERCULES INCORPORATED ou bien sous la dénomination de "PD 170" ou "DELSETTE 101" par la Société HERCULES dans le cas du copolymère d'acide adipique/époxypropyl/diéthylène-triamine.
(5) Les cyclohomopolymères de méthyl diallyl amine ou de diméthyl diallyl ammonium tels que les homopolymères comportant comme constituant principal de la chaîne des motifs répondant aux formules (I) ou (II) : 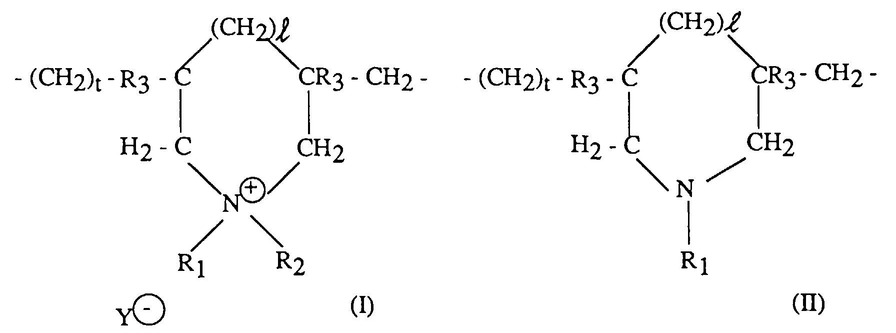
   ℓ et t sont égaux à 0 ou 1, et la somme ℓ + t = 1;
   R₃ désigne hydrogène ou méthyle;
   R₁ et R₂ désignent, indépendamment l'un de l'autre, un groupement alkyle ayant de 1 à 22 atomes de carbone, un groupement hydroxyalkyle dans lequel le groupement alkyle a de préférence 1 à 5 atomes de carbone, un groupement amidoalkyle inférieur et où R₁ et R₂ peuvent désigner conjointement avec l'atome d'azote auquel ils sont rattachés, des groupements hétérocycliques tels que pipéridinyle ou morpholinyle;
   Y^{⊖} est un anion tel que bromure, chlorure, acétate, borate, citrate, tartrate, bisulfate, bisulfite, sulfate, phosphate.

   Parmi les polymères définis ci-dessus, on peut citer plus particulièrement l'homopolymère de chlorure de diméthyldiallylammonium vendu sous la dénomination "MERQUAT 100" par la Société MERCK, ayant un poids moléculaire inférieur à 100 000.
   Ces polymères sont décrits plus particulièrement dans le brevet français 2.080.759, et son certificat d'addition n° 2.190.406.
(6) Le polymère de polyammonium quatenaire contenant des motifs récurrents répondant à la formule : 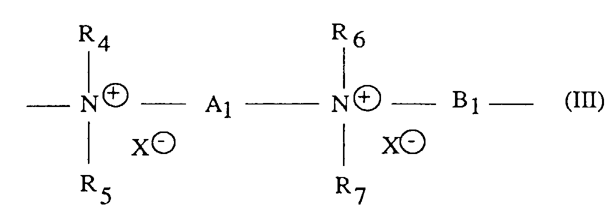 dans laquelle R₄ et R₅, R₆ et R₇ étant identiques ou différents, représentent des radicaux aliphatiques, alicycliques ou arylaliphatiques contenant de 1 à 20 atomes de carbone ou des radicaux hydroxy alkylaliphatiques inférieurs, ou bien R₄ et R₅ et R₆ et R₇ ensemble ou séparément, constituent avec les atomes d'azote auxquels ils sont rattachés, des hétérocycles contenant éventuellement un second hétéroatome autre que l'azote ou bien R₄, R₅, R₆ et R₇ représentent un radical alkyle en C₁-C₆ linéaire ou ramifié substitué par un groupement nitrile, ester, acyle, amide ou 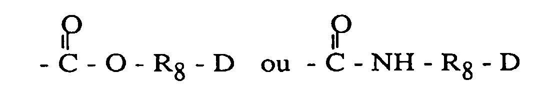 où R₈ est un alkylène et D un groupement ammonium quaternaire.
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X^{⊖} désigne un anion dérivé d'un acide minéral ou organique.
   A₁ et R₄ et R₆ peuvent former avec les deux atomes d'azote auxquels ils sont rattachés, un cycle pipérazinique; en outre, si A₁ désigne un radical alkylène ou hydroxyalkylène linéaire ou ramifié, saturé ou insaturé, B₁ peut également désigner un groupement

      (CH₂)ₙ -CO - D - OC - (CH₂)ₙ -
   dans lequel D désigne :
   a) un reste de glycol de formule : - O - Z - O -
      où Z désigne un radical hydrocarboné linéaire ou ramifié ou un groupement répondant aux formules : 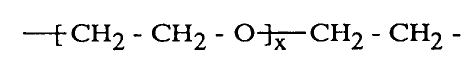 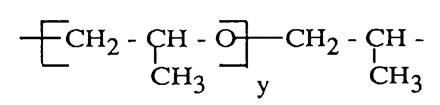 où x et y désignent un nombre entier de 1 à 4, représentant un degré de polymérisation défini et unique ou un nombre quelconque de 1 à 4 représentant un degré de polymérisation moyen;
   b) un reste de diamine bis-secondaire tel qu'un dérivé de pipérazine;
   c) un reste de diamine bis-primaire de formule :

      - NH - Y - NH -

      où Y désigne un radical hydrocarboné linéaire ou ramifié, ou bien le radical bivalent

      - CH₂ - CH₂ - S - S - CH₂ - CH₂ -
   d) un groupement uréylène de formule :

      - NH - CO - NH - ;

      X^{⊖} est un anion tel que chlorure ou bromure.

   Ces polymères ont une masse moléculaire généralement comprise entre 1000 et 100 000.
   Des polymères de ce type sont décrits en particulier dans les brevets français 2 320 330, 2 270 846, 2 316 271, 2 336 434, et 2 413 907 et les brevets US-A-2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.
(7) Les polymères de polyammonium quaternaires constitués de motifs de formule (IV) : 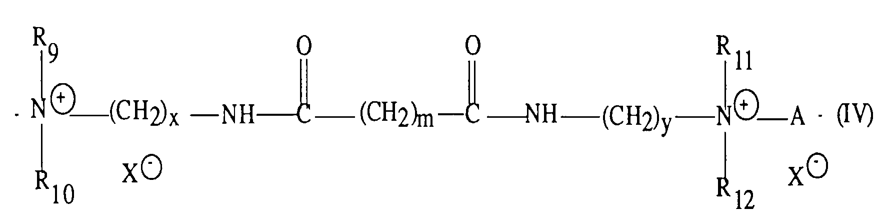 dans laquelle :
   R₉, R₁₀, R₁₁ et R₁₂, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à O ou un nombre entier compris entre 1 et 6, sous réserve que R₉, R₁₀, R₁₁ et R₁₂ ne représentent pas simultanément un atome d'hydrogène;
   x et y, identiques ou différents, sont des nombres entiers compris entre 1 et 6;
   m est égal à O ou à un nombre entier compris entre 1 et 34;
   x désigne un atome d'halogène;
   A désigne un radical d'un dihalogénure et représente de préférence

      - CH₂ - CH₂ - O - CH₂ - CH₂ -

De tels composés sont décrits plus en détail dans la demande EP-A-122 324.

On peut par exemple citer parmi ceux-ci, les produits "MIRAPOL A 15", "MIRAPOL AD1", "MIRAPOL AZ1", "MIRAPOL 175", vendus par la Société MIRANOL.

Les céramides et/ou glycocéramides sont connus en elles-mêmes et sont des molécules naturelles ou synthétiques répondant à la formule générale : 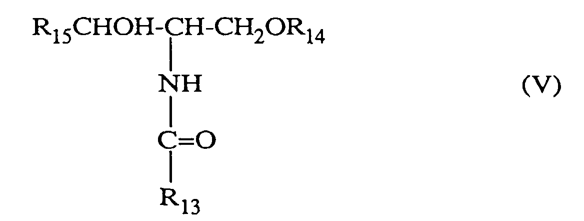 dans laquelle :
R₁₃ désigne un radical alkyle, linéaire ou ramifié, saturé ou insaturé, dérivé d'acides gras en C₁₄-C₃₀, ce radical pouvant être substitué par un groupement hydroxyle en position α ou un groupement hydroxyle en position ω estérifié par un acide gras saturé ou insaturé en C₁₆-C₃₀;
R₁₄ désigne un atome d'hydrogène ou un radical (glycosyle)ₙ, (galactosyle)ₘ ou sulfogalactosyle;
dans lesquels :
n est un entier variant de 1 à 4; et
m est un entier variant de 1 à 8;
R₁₅ désigne un radical hydrocarboné en C₁₅-C₂₆, saturé ou insaturé en position α, pouvant être substitué par un ou plusieurs radicaux alkyle en C₁-C₁₄;
dans le cas des céramides ou glycocéramides naturelles, R₁₅ peut également désigner un radical α-hydroxyalkyle en C₁₅-C₂₆, le groupement hydroxyle étant éventuellement estérifié par un α-hydroxyacide en C₁₆-C₃₀.

Les céramides préférées sont celles décrites par Downing dans Arch. Dermatol, Vol. 123, 1381-1384, 1987, ou celles décrites dans le brevet français FR-2 673 179, dont les structures peuvent être les suivantes :

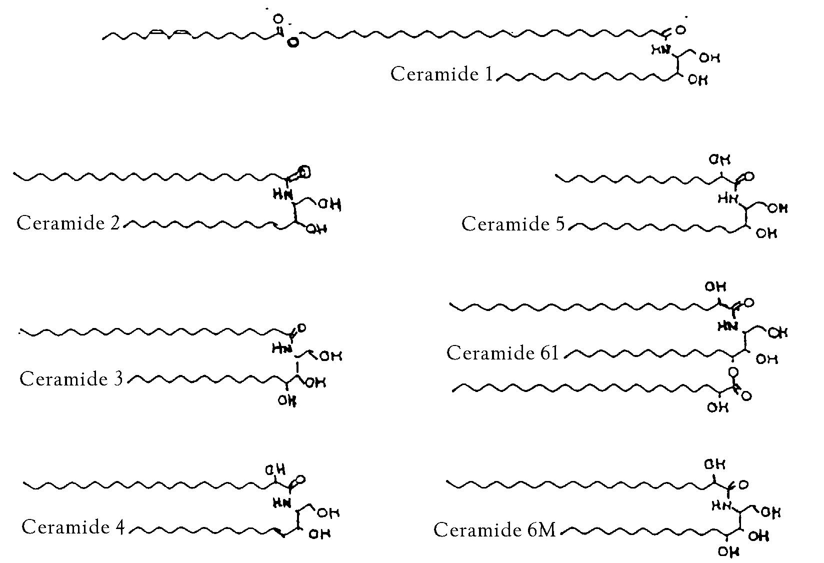

Les céramides plus particulièrement préférés sont les composés de formule pour lesquels :
R₁₃ désigne un alkyle saturé ou insaturé, dérivé d'acide gras en C₁₆-C₂₂;
R₁₄ désigne hydrogène;
R₁₅ désigne un radical linéaire saturé en C₁₅.

De tels composés sont par exemple :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
ou les mélanges de ces composés.

On utilise également de préférence ceux pour lesquels :
R₁₃ désigne un radical alkyle saturé ou insaturé, dérivé d'acide gras;
R₁₄ désigne galactosyle ou sulfogalactosyle; et
R₁₅ désigne -CH=CH-(CH₂)₁₂-CH₃.

On peut citer le produit constitué d'un mélange de ces composés, vendu sous la dénomination commerciale GLYCOCER par la Société WAITAKI INTERNATIONAL BIOSCIENCES.

Les polymères cationiques sont utilisés de préférence dans des proportions de 0,05 à 5% en poids exprimées en matière active, et de préférence entre 0,1 et 3% en poids par rapport au poids total de la composition.

Les céramides et/ou glycocéramides sont utilisés de préférence dans des proportions de 0,005% et 5% en poids exprimées en matière active, et de préférence entre 0,01 et 3% en poids par rapport au poids total de la composition.

Ces compositions peuvent contenir des agents tensio-actifs, tels que des tensio-actifs non-ioniques ou cationiques dans des proportions généralement comprises entre 0,1 et 10% en poids.

Des agents tensio-actifs non-ioniques utilisés dans une forme de réalisation préférée de l'invention sont connus en eux-mêmes et peuvent être choisis parmi les alcools, les alphadiols, les alkylphénols, les acides gras polyéthoxylés, polypropoxylés ou polyglycérolés à chaîne grasse comportant 8 à 18 atomes de carbone, le nombre de groupements d'oxyde d'éthylène et d'oxyde de propylène pouvant aller en particulier de 2 à 50 et le nombre de groupements glycérol pouvant aller notamment de 2 à 30.

On peut également citer les copolymères d'oxyde d'éthylène et de propylène, les condensats d'oxyde d'éthylène et de propylène sur des alcools gras; les amides gras polyéthoxylés ayant de préférence 2 à 30 moles d'oxyde d'éthylène, les amides gras polyglycérolés comportant en moyenne 1 à 5 groupements glycérol et en particulier 1,5 à 4 groupements glycérol; les amines grasses polyéthoxylées ayant de préférence 2 à 3 moles d'oxyde d'éthylène; les esters d'acides gras du sorbitan oxyéthylénés ayant de 2 à 30 moles d'oxyde d'éthylène, les esters d'acides gras du sucrose, les esters d'acides gras du polyéthylèneglycol, les alkylpolyglycosides, les dérivés de N-alkyl glucamine, les oxydes d'amines tels que les oxydes d'alkyl(C₁₀-C₁₄) amines ou les oxydes de N-acylaminopropylmorpholine. Les alkyl polyglycosides et les alcools alphadiols alkylphénols d'acides gras polyglycérolés sont plus particulièrement préférés.

Les compositions conformes à l'invention peuvent contenir des agents tensio-actifs cationiques comme les sels d'amines grasses primaires, secondaires ou tertiaires, éventuellement polyoxyéthylénés; les sels d'ammonium quaternaires tels que les chlorures ou les bromures de tétraalkylammonium, d'alkylamidoalkyltrialkyl ammonium, de trialkylbenzylammonium, de trialkylhydroxyalkyl ammonium ou d'alkylpyridinium, les dérivés d'imidazoline.

Les compositions peuvent contenir des agents épaississants tels que l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxy propylméthylcellulose, la gomme de guar ou ses dérivés, les gommes de xanthane, les scléroglucanes, les acides polyacryliques réticulés, les polyuréthanes, les copolymères à base d'acide ou d'anhydride maléique éventuellement réticulé, des épaississants associatifs porteurs de chaînes grasses de type naturel, comme le produit commercialisé sous la dénomination NATROSOL PLUS ou synthétiques, comme les produits commercialisés sous la dénomination PEMULEN.

L'épaississement peut également être obtenu par mélange du polyéthylèneglycol et de stéarates ou de distéarates de polyéthylène glycol ou par mélange d'esters phosphoriques et d'amides.

Le milieu cosmétiquement acceptable est de préférence constitué par de l'eau ou un mélange d'eau et de solvants cosmétiquement acceptables, tels que des monoalcools, des polyalcools, des éthers de glycol ou des esters d'acides gras utilisés seuls ou en mélange.

On peut citer plus particulièrement les alcools inférieurs tels que l'éthanol, l'isopropanol, les polyalcools tels que le glycérol, le diéthylèneglycol, les éthers de glycol, les alkyléthers de glycol ou de diéthylèneglycol.

Le pH de ces compositions est généralement compris entre 2 et 9, et en particulier entre 3 et 8. Il est ajusté par des agents alcalinisants ou acidifiants cosmétiquement acceptables et connus en eux-mêmes.

Les compositions conformes à l'invention peuvent contenir des agents conservateurs, des séquestrants, des adoucissants, des modificateurs de mousse, des acidifiants ou alcalinisants, des parfums, des colorants, des agents modificateurs de viscosité, des agents nacrants, des agents hydratants, des agents antipelliculaires, des agents antiséborrhéiques, des filtres solaires, des protéines, des vitamines, des hydroxyacides, des sels, des parfums, des agents conservateurs, des adoucissants, des modificateurs de mousse, des détoxifiants, des agents réducteurs ou fixateurs de permanente ou leurs mélanges.

On peut utiliser en plus d'autres agents conditionneurs. On peut citer à cet effet les huiles naturelles hydrogénées ou non, synthétiques ou non, hydrocarbonées, cycliques ou aliphatiques, linéaires ou ramifiées, saturées ou non, solubles ou non, des alcools gras; des silicones volatiles ou non, organo modifiées ou non, solubles dans le milieu ou non non; des huiles perfluorées ou fluorées, les polybutènes et polyisobutènes, les esters gras se présentant sous forme liquide, pâteuse ou solide, les esters d'alcools polyhydriques, des glycérides, des cires naturelles ou synthétiques, des gommes et résines de silicones; des protéines ou le mélange de ces différents agents.

Les compositions conformes à l'invention peuvent être utilisées avant ou après shampooing, avant ou après une permanente ou entre le temps de la réduction et de la fixation, avant ou après décoloration ou coloration ou défrisage. Elles peuvent également être utilisées pour la coloration des fibres kératiniques telles que les cheveux, auquel cas elles contiennent des colorants d'oxydation et/ou des colorants directs bien connus dans le domaine de la teinture des cheveux. Des colorants de ce type sont décrits notamment dans Charles ZVIAK "Sciences des Traitements capillaires " Ed. Masson, 1988. Ces compositions peuvent également être utilisées pour des permanentes, auquel cas elles contiennent des agents réducteurs ou des agents fixateurs ou neutralisants, suivant que la composition est utilisée pour la réduction ou la fixation des cheveux. De tels produits sont décrits notamment dans Charles ZVIAK "Sciences des Traitements capillaires" mentionné ci-dessus.

Elles se présentent généralement sous forme d'émulsions ou dispersions ou de solutions.

Elles peuvent également être sous forme de liquides fluides ou épaissis, de gels ou de crèmes.

Elles peuvent être utilisées en l'état ou être diluées avant utilisation.

Elles peuvent également être conditionnées dans un récipient sous pression et être délivrées sous forme de spray, de liquides, de crèmes, de gels ou de mousses.

Elles peuvent être rincées ou non.

Un autre objet de l'invention est constitué par le procédé de traitement des cheveux, consistant à appliquer une composition telle que définie ci-dessus, sur les cheveux à traiter et à procéder éventuellement au rinçage.

Les exemples qui suivent sont destinés à illustrer l'invention sans pour autant présenter un caractère limitatif.

### EXEMPLES

### EXEMPLE 1

On prépare la composition suivante :
- Polyquaternium 2 (CTFA), vendu sous la dénomination MIRAPOL A 15 1 g MA
- Céramide A 0,5 g
- APG 300 est un alkyl(C₉/C₁₀/C₁₁) polyglycoside (1,4), vendu en solution aqueuse à 50% de matière active (MA) par la Société HENKEL 2 g
- Hydroxyéthylcellulose 0,5 g
- Eau qsp 100 g
- pH ajusté à 7,5 par NaOH

| | |
|---|---|
| Schéma de pressurisation : | 90 g de composition |
| | 10 g Aérogaz 3-2 |

Cette composition forme à la sortie du dispositif aérosol une mousse qui est appliquée sur les cheveux, elle disparait après massage.

### Céramide A :

N-oléoyldihydrosphingosine de formule : 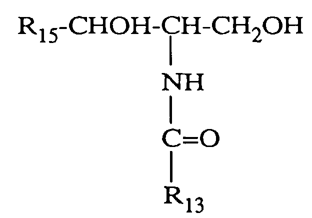 dans laquelle :
R₁₅ = C₁₅H₃₁
R₁₅ = C₁₇H₃₃

### Polymère A :

Polymère comportant des motifs de formule : 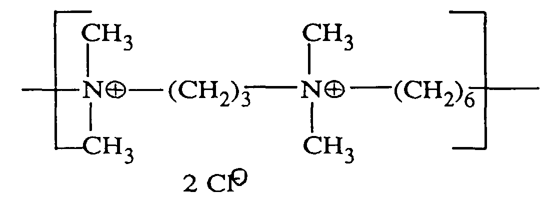 préparé et décrit dans le brevet français n° 2270846.

### EXEMPLES 2 à 5

| EXEMPLE | 2 | 2A | 2B |
|---|---|---|---|
| Céramide A | 0,2 g | 0,7 g | - |
| Polymère A | 0,5 g | - | 0,7 g |
| Propanol-2 | 60 g | 60 g | 60 g |
| Eau qsp | 100 g | 100 g | 100 g |

Les exemples 2A et 2B ne sont pas conformes à l'invention.

On applique 5 g des compositions sur des mèches de 2,5 g préalablement lavées et rincées. On laisse pauser 5 minutes, puis on rince à l'eau courante. On évalue ensuite le démêlage des cheveux humides.

On constate que le démêlage pour la composition 2 est nettement supérieur au démêlage obtenu avec les compositions 2A et 2B contenant uniquement, soit la céramide, soit le polymère cationique.

Des résultats similaires ont été obtenus en utilisant à la place du polymère A, les polymères suivants : MIRAPOL A 15, MERQUAT 100, et un polycondensat de diéthylènediamine et d'acide adipique réticulé avec l'épichlorhydrine, décrit dans le brevet français FR-2.252.840.

MERQUAT 100 (CALGON) = homopolymère du chlorure de diméthyl diallyl ammonium (polyquaternium-6).

### EXEMPLE 6

On a préparé la composition suivante :
- Dichlorhydrate de 1-amino 4-β-méthoxyéthylaminobenzène 0,71 g
- Dichlorhydrate de 1-hydroxyéthyloxy 2,4-diaminobenzène 0,72 g
- Alcool cétylique et stéarylique en mélange 50/50 18 g
- 2-octyl dodécanol 3 g
- Alcool cétylstéarylique oxyéthyléné à 15 moles d'oxyde d'éthylène 3 g
- Laurylsulfate d'ammonium à 30% de MA (3,6 g MA) 12 g
- N-oléoyl dihydrosphingosine 0,3 g
- Polymère constitué de motifs de formule : 3 g 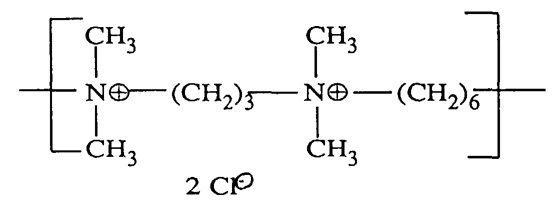
pouvant être préparé comme décrit dans les
brevets BF 2 270 846 et 2 333 012
- Ammoniaque à 22° Bé 12 g
- Bisulfite de sodium à 35° Bé 1,5 g
- Parfum qs
- Eau déminéralisée qsp 100 g

Cette composition tinctoriale est une crème que l'on mélange avec 1,5 fois son poids d'eau oxygénée à 20 volumes.

Le mélange crémeux obtenu est appliqué 30 minutes sur des cheveux gris à 90% de blancs.

Après rinçage et shampooing, on obtient une nuance bleue.

### EXEMPLE 7

On a préparé la composition réductrice suivante :
- Acide thiolactique 8 g
- Ammoniaque qs pH=7
- Bicarbonate d'ammonium 6,5 g
- Agent séquestrant 0,2 g
- N-oléoyl dihydrosphingosine 0,1 g
- Polymère constitué de motifs de formule : 3 g 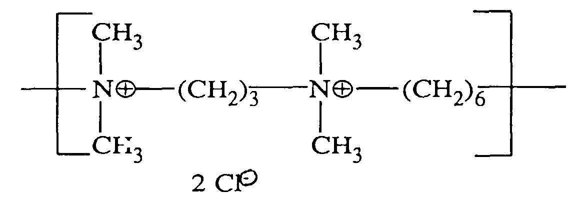 pouvant être préparé comme décrit dans les
   brevets BF 2 270 846 et 2 333 012
   - Alkyl C₉/C₁₀/C₁₁ polyglycoside (1,4) (APG 300) 3,5 g MA
   - Parfum qs
   - Eau déminéralisée qsp 100 g

On enroule des cheveux sur des rouleaux ayant un diamètre variant de 4 mm à 10 mm, puis on laisse agir la composition pendant un temps de pause de 10 à 15 minutes.

Après rinçage, on applique ensuite sur les cheveux réduits une composition oxydante à base d'eau oxygénée à 8 volumes et à pH 4.

On laisse agir cette composition oxydante pendant 5 minutes, puis on rince les cheveux et on enlève alors les rouleaux. Les cheveux présentent alors de belles boucles.

## Revendications

1. Composition destinée au traitement et à la protection des cheveux, caractérisée par le fait qu'elle contient dans un milieu cosmétiquement acceptable, au moins un céramide et/ou glycocéramide et au moins un polymère cationique comportant des groupements amine primaires, secondaires, tertiaires ou ammonium quaternaire dans la chaîne principale et ayant une viscosité à 1% en poids de matière active dans de l'eau inférieure à 15 m Pa.s, la composition contenant moins de 4% en poids d'agents tensio-actifs anioniques et/ou amphotères et/ou zwittérioniques.

2. Composition selon la revendication 1, caractérisée par le fait que les polymères cationiques sont choisis parmi :
(1) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères;
(2) les polyaminoamides solubles dans l'eau, éventuellement réticulés et/ou alcoylés;
(3) les dérivés de polyaminoamides résultant de la condensation de polyalcoylène polyamines avec des acides polycarboxyliques suivie d'une alcoylation par des agents bifonctionnels;
(4) les polymères obtenus par réaction d'une polyalkylène polyamine comportant deux groupements amine primaire et au moins un groupement amine secondaire avec un acide dicarboxylique;
(5) les cyclohomopolymères de méthyl diallyl amine ou de diméthyl diallyl ammonium;
(6) les polymères de polyammonium quaternaires.

3. Composition selon l'une quelconque des revendications 1 ou 2, caractérisée par le fait que les céramides et/ou glycocéramides sont choisis parmi les composés de formule générale : 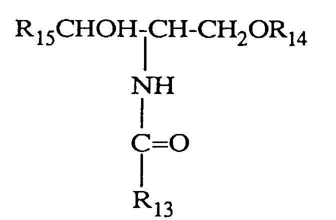 dans laquelle :
R₁₃ désigne un radical alkyle, linéaire ou ramifié, saturé ou insaturé, dérivé d'acides gras en C₁₄-C₃₀, ce radical pouvant être substitué par un groupement hydroxyle en position α ou un groupement hydroxyle en position ω estérifié par un acide gras saturé ou insaturé en C₁₆-C₃₀;
R₁₄ désigne un atome d'hydrogène ou un radical (glycosyle)ₙ, (galactosyle)ₘ ou sulfogalactosyle;
où n est un entier variant de 1 à 4; et m est un entier variant de 1 à 8;
R₁₅ désigne un radical hydrocarboné en C₁₅-C₂₆, saturé ou insaturé en position α, pouvant être substitué par un ou plusieurs radicaux alkyle en C₁-C₁₄;
dans le cas des céramides ou glycocéramides naturels, R₁₅ peut également désigner un radical α-hydroxyalkyle en C₁₅-C₂₆, un groupement hydroxyle étant éventuellement estérifié par un α-hydroxyacide en C₁₆-C₃₀.

4. Composition selon l'une quelconque des revendications 1 à 3, caractérisée par le fait que les polymères cationiques sont présents dans des proportions comprises entre 0,05 et 5% en poids exprimées en matière active et de préférence entre 0,1 et 3% en poids par rapport au poids total de la composition.

5. Composition selon l'une quelconque des revendications 1 à 3, caractérisée par le fait que les céramides et/ou glycocéramides sont présentes dans des proportions comprises entre 0,005 et 5% en poids exprimées en matière active, et de préférence entre 0,01 et 3% en poids par rapport au poids total de la composition.

6. Composition selon l'une quelconque des revendications 1 à 5, caractérisée par le fait que le pH des compositions est compris entre 2 et 9.

7. Composition selon l'une quelconque des revendications 1 à 6, caractérisée par le fait que la composition contient également des agents tensio-actifs non-ioniques et/ou cationiques.

8. Composition selon l'une quelconque des revendications 1 à 7, caractérisée par le fait que la composition contient des agents épaississants.

9. Composition selon l'une quelconque des revendications 1 à 8, caractérisée par le fait que le milieu cosmétiquement acceptable est constitué par de l'eau, un mélange d'eau et de solvants cosmétiquement acceptables choisis parmi les monoalcools, les polyalcools, les éthers de glycol ou les esters d'acides gras, utilisés seuls ou en mélange.

10. Composition selon l'une quelconque des revendications 1 à 9, caractérisée par le fait que la composition contient également des agents conservateurs, des agents séquestrants, adoucissants, modificateurs de mousse, des colorants, des agents modificateurs de viscosité, des agents nacrants, des agents hydratants, antipelliculaires, antiséborrhéiques, des filtres solaires, des protéines, des vitamines, des α-hydroxyacides, des sels, des détoxifiants, des parfums, des agents réducteurs ou des agents fixateurs de permanente, ou leurs mélanges.

11. Composition selon l'une quelconque des revendications 1 à 10, caractérisée par le fait que la composition contient également d'autres agents conditionneurs choisis parmi les huiles naturelles hydrogénées ou non, synthétiques ou non, cycliques ou aliphatiques, linéaires ou ramifiées, saturées ou non, des alcools gras; les silicones volatiles ou non, organomodifiées ou non, solubles dans le milieu ou non; les huiles fluorées ou perfluorées; des polybutènes, des polyisobutènes; des esters gras, des esters d'alcools polyhydriques; des glycérides; des cires synthétiques ou naturelles, des gommes et résines de silicones; des protéines ou le mélange de ces différents agents.

12. Composition selon l'une quelconque des revendications 1 à 11, caractérisée par le fait qu'elle se présente sous forme de liquide fluide ou épaissi, de gel ou de crème, de mousse, éventuellement conditionnée sous pression.

13. Procédé non lavant de traitement et de protection des cheveux, caractérisé par le fait que l'on applique une composition telle que définie dans l'une quelconque des revendications 1 à 12 sur les cheveux et qu'après un éventuel temps de pose, on procède éventuellement au rinçage.

14. Utilisation de la composition telle que définie dans l'une quelconque des revendications 1 à 12, pour le traitement non lavant des cheveux.

15. Utilisation de la composition selon l'une quelconque des revendications 1 à 12, pour la coloration des cheveux.

16. Utilisation de la composition selon l'une quelconque des revendications 1 à 12, pour la réalisation d'une permanente des cheveux.

## Claims

1. Composition intended for the treatment and protection of hair, characterized in that it contains, in a cosmetically acceptable medium, at least one ceramide and/or glycoceramide and at least one cationic polymer containing primary, secondary or tertiary amine groups or quaternary ammonium groups in the main chain and having a viscosity at a concentration of 1 % by weight of active substance in water of less than 15 mPa.s, the composition containing less than 4 % by weight of anionic and/or amphoteric and/or zwitterionic surfactants.

2. Composition according to Claim 1, characterized in that the cationic polymers are chosen from:
(1) polymers consisting of piperazinyl units and bivalent alkylene or hydroxyalkylene radicals having unbranched or branched chains, optionally interrupted by oxygen, sulphur or nitrogen atoms or by aromotic or hererocyclic rings, as well as the oxidation and/or quaternization products of these polymers;
(2) water-soluble polyaminoamides, optionally crosslinked and/or alkylated;
(3) polyaminoamide derivatives resulting from the condensation of polyalkylenepolyamines with polycarboxylic acids, followed by an alkylation with bifunctional agents;
(4) polymers obtained by reacting a polyalkylenepolyamine containing two primary amine groups and at least one secondary amine group with a dicarboxylic acid;
(5) methyldiallylamine or dimethyldiallylammonium cyclohomopolymers;
(6) poly(quaternary ammonium) polymers.

3. Composition according to either of Claims 1 and 2, characterized in that the ceramides and/or glycoceramides are chosen from the compounds of general formula: 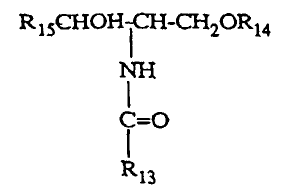 in which:
R₁₃ denotes a saturated or unsaturated, linear or branched alkyl radical derived from C₁₄-C₃₀ fatty acids, it being possible for this radical to be substituted with a hydroxyl group at the α-position or a hydroxyl group at the ω-position esterified with a saturated or unsaturated C₁₆-C₃₀ fatty acid;
R₁₄ denotes a hydrogen atom or a (glycosyl)ₙ, (galactosyl)ₘ or sulphogalactosyl radical;
where n is an integer varying from 1 to 4; and m is an integer varying from 1 to 8;
R₁₅ denotes a C₁₅-C₂₆ hydrocarbon radical, saturated or unsaturated at the α-position and which can be substituted with one or more C₁-C₁₄ alkyl radicals;
in the case of natural ceramides or glycoceramides, R₁₅ can also denote a C₁₅-C₂₆ α-hydroxyalkyl radical, a hydroxyl group being optionally esterified with a C₁₆-C₃₀ α-hydroxy acid.

4. Composition according to any one of Claims 1 to 3, characterized in that the cationic polymers are present in proportions of between 0.05 and 5 % by weight expressed as active substance, and preferably between 0.1 and 3 % by weight, relative to the total weight of the composition.

5. Composition according to any one of Claims 1 to 3, characterized in that the ceramides and/or glycoceramides are present in proportions of between 0.005 and 5 % by weight expressed as active substance, and preferably between 0.01 and 3 % by weight, relative to the total weight of the composition.

6. Composition according to any one of Claims 1 to 5, characterized in that the pH of the compositions is between 2 and 9.

7. Composition according to any one of Claims 1 to 6, characterized in that the composition also contains nonionic and/or cationic surfactants.

8. Composition according to any one of Claims 1 to 7, characterized in that the composition contains thickening agents.

9. Composition according to any one of Claims 1 to 8, characterized in that the cosmetically acceptable medium consists of water or a mixture of water and cosmetically acceptable solvents chosen from monohydric alcohols, polyhydric alcohols, glycol ethers or fatty acid esters, used alone or mixed.

10. Composition according to any one of Claims 1 to 9, characterized in that the composition also contains preservatives, sequestering agents, emollients, foam modifiers, colorants, viscosity modifiers, pearlescent agents, hydrating agents, antidandruff agents, antiseborrhoeic agents, sunscreen agents, proteins, vitamins, α-hydroxy acids, salts, detoxifying agents, perfumes, permanent-waving reducing or fixing agents or mixtures thereof.

11. Composition according to any one of Claims 1 to 10, characterized in that the composition also contains other conditioning agents chosen from natural, hydrogenated or unhydrogenated, synthetic or non-synthetic oils which are cyclic or aliphatic, linear or branched and saturated or unsaturated, fatty alcohols; volatile or non-volatile silicones, organomodified or otherwise and which are soluble or insoluble in the medium; fluorinated or perfluorinated oils; polybutenes, polyisobutenes; fatty esters, esters of polyhydric alcohols; glycerides; synthetic or natural waxes, silicone gums and resins; proteins; or a mixture of these different agents.

12. Composition according to any one of Claims 1 to 11, characterized in that it takes the form of a fluid or thickened liquid, a gel or cream or a foam, optionally packaged under pressure.

13. Non-washing process for the treatment and protection of hair, characterized in that a composition as defined in any one of Claims 1 to 12 is applied to the hair, and in that, after an optional period of exposure, rinsing is optionally carried out.

14. Use of the composition as defined in any one of Claims 1 to 12, for the non-washing treatment of hair.

15. Use of the composition according to any one of Claims 1 to 12, for the dyeing of hair.

16. Use of the composition according to any one of Claims 1 to 12, for carrying out permanent-waving of hair.

## Patentansprüche

1. Zusammensetzung zur Behandlung und zum Schutz des Haares, dadurch gekennzeichnet, daß sie in einem kosmetisch akzeptablen Medium mindestens ein Ceramid und/oder Glykoceramid und mindestens ein kationisches Polymer enthält, das primäre, sekundäre oder tertiäre Aminogruppen oder quartäre Ammoniumgruppen in der Hauptkette enthält und bei 1 Gew.-% Wirkstoff in Wasser eine Viskosität unter 15 mPa·s aufweist, wobei die Zusammensetzung weniger als 4 Gew.-% anionische und/oder amphotere und/oder zwitterionische grenzflächenaktive Stoffe enthält.

2. Zusammensetzung nach Anspruch 1, dadurch gekennzeichnet, daß die kationischen Polymere ausgewählt sind unter:
(1) den Polymeren, die aus Piperazinyleinheiten und zweiwertigen Alkylen- oder Hydroxyalkylengruppen mit geraden oder verzweigten Ketten bestehen, die gegebenenfalls durch Sauerstoffatome, Schwefelatome, Stickstoffatome oder aromatische oder heterocyclische Ringe unterbrochen sind, sowie den Oxidationsprodukten und/oder Quaternisierungsprodukten dieser Polymere;
(2) den wasserlöslichen Polyaminoamiden, die gegebenenfalls vernetzt und/oder alkyliert sind;
(3) den Polyaminoamidderivaten, die aus der Kondensation von Polyalkylenpolyaminen mit Polycarbonsäuren und anschließender Alkylierung mit bifunktionellen Mitteln resultieren;
(4) den Polymeren, die durch Umsetzung eines Polyalkylenpolyamins, das zwei primäre Aminogruppen und mindestens eine sekundäre Aminogruppe aufweist, mit einer Dicarbonsäure hergestellt werden;
(5) den Methyldiallylamin- oder Dimethyldiallylammonium-Cyclohomopolymeren; und
(6) den quartären Polyammoniumpolymeren.

3. Zusammensetzung nach einem der Ansprüche 1 oder 2, dadurch gekennzeichnet, daß die Ceramide und/oder Glykoceramide unter den Verbindungen der folgenden allgemeinen Formel ausgewählt sind: 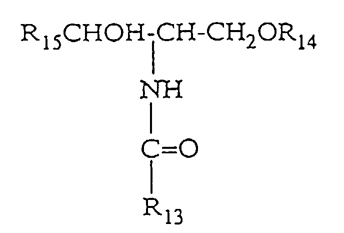 worin bedeuten:
R₁₃ eine geradkettige oder verzweigte, gesättigte oder ungesättigte, von C₁₄₋₃₀-Fettsäuren abgeleitete Alkylgruppe, wobei die Gruppe in α-Stellung mit einer Hydroxygruppe oder in ω-Stellung mit einer Hydroxygruppe substituiert sein kann, die mit einer gesättigten oder ungesättigten C₁₆₋₃₀-Fettsäure verestert ist;
R₁₄ Wasserstoff oder eine Gruppe (Glykosyl)ₙ, (Galactosyl)ₘ oder Sulfogalactosyl, worin n eine ganze Zahl von 1 bis 4 und m eine ganze Zahl von 1 bis 8 bedeutet;
R₁₅ eine gesättigte oder in α-Stellung ungesättigte C₁₅₋₂₆-Kohlenwasserstoffgruppe, die mit einer oder mehreren C₁₋₁₄-Alkylgruppen substituiert sein kann;
wobei im Falle von natürlichen Ceramiden oder Glykoceramiden die Gruppe
R₁₅ auch eine C₁₅₋₂₆-α-Hydroxyalkylgruppe bedeuten kann, wobei die Hydroxygruppe gegebenenfalls mit einer C₁₆₋₃₀-α-Hydroxysäure verestert ist.

4. Zusammensetzung nach einem der Ansprüche 1 bis 3, dadurch gekennzeichnet, daß die kationischen Polymere in als Wirkstoff ausgedrückten Mengenanteilen im Bereich von 0,05 bis 5 Gew.-% und vorzugsweise 0,1 bis 3 Gew.-%, bezogen auf das Gesamtgewicht der Zusammensetzung, vorliegen.

5. Zusammensetzung nach einem der Ansprüche 1 bis 3, dadurch gekennzeichnet, daß die Ceramide und/oder Glykoceramide in als Wirkstoff ausgedrückten Mengenanteilen im Bereich von 0,005 bis 5 Gew.-% und vorzugsweise 0,01 bis 3 Gew.-%, bezogen auf das Gesamtgewicht der Zusammensetzung, vorliegen.

6. Zusammensetzung nach einem der Ansprüche 1 bis 5, dadurch gekennzeichnet, daß der pH-Wert der Zusammensetzung im Bereich von 2 bis 9 liegt.

7. Zusammensetzung nach einem der Ansprüche 1 bis 6, dadurch gekennzeichnet, daß die Zusammensetzung ferner nichtionische und/oder kationische grenzflächenaktive Stoffe enthält.

8. Zusammensetzung nach einem der Ansprüche 1 bis 7, dadurch gekennzeichnet, daß die Zusammensetzung Verdickungsmittel enthält.

9. Zusammensetzung nach einem der Ansprüche 1 bis 8, dadurch gekennzeichnet, daß das kosmetisch akzeptable Medium aus Wasser und einem Gemisch von Wasser und kosmetisch akzeptablen Lösungsmitteln besteht, die unter den Monoalkoholen, Polyalkoholen, Glykolethern oder Fettsäureestern ausgewählt sind und die einzeln oder im Gemisch eingesetzt werden.

10. Zusammensetzung nach einem der Ansprüche 1 bis 9, dadurch gekennzeichnet, daß die Zusammensetzung ferner Konservierungsmittel, Maskierungsmittel, Mittel für die Geschmeidigkeit, Schaummodifikatoren, Färbemittel, Viskositätsmodifikatoren, Perlglanzmittel, Hydratisierungsmittel, Mittel gegen Schuppen, Antiseborrhöika, Sonnenschutzfilter, Proteine, Vitamine, α-Hydroxysäuren, Salze, Detoxikationsmittel, Parfüms, Reduktionsmittel oder Fixiermittel für Dauerwellen oder deren Gemische enthält.

11. Zusammensetzung nach einem der Ansprüche 1 bis 10, dadurch gekennzeichnet, daß die Zusammensetzung ferner weitere Konditioniermittel enthält, die unter den hydrierten oder nicht hydrierten, synthetischen oder nicht synthetischen, cyclischen oder aliphatischen, geradkettigen oder verzweigten, gesättigten oder ungesättigten, natürlichen Ölen, Fettalkohlen; flüchtigen oder nicht flüchtigen, gegebenenfalls organomodifizierten, in dem Medium löslichen oder unlöslichen Siliconen; fluorierten oder perfluorierten Ölen; Polybutenen, Polyisobutenen; Fettsäureestern, Estern von mehrwertigen Alkoholen; Glyceriden; synthetischen oder natürlichen Wachsen, Silicongummis und Siliconharzen; Proteinen oder den Gemischen dieser verschiedenen Mittel ausgewählt sind.

12. Zusammensetzung nach einem der Ansprüche 1 bis 11, dadurch gekennzeichnet, daß sie gegebenenfalls unter Druck konfektioniert als fluide oder dickflüssige Flüssigkeit, Gel, Creme oder Schaum vorliegt.

13. Nicht reinigendes Verfahren zur Behandlung und zum Schutz der Haare, dadurch gekennzeichnet, daß eine Zusammensetzung nach einem der Ansprüche 1 bis 12 auf das Haar aufgetragen und anschließend gegebenenfalls nach einer Einwirkungszeit gegebenenfalls gespült wird.

14. Verwendung der Zusammensetzung nach einem der Ansprüche 1 bis 12 zur nicht reinigenden Behandlung des Haares.

15. Verwendung der Zusammensetzung nach einem der Ansprüche 1 bis 12 zum Färben von Haaren.

16. Verwendung der Zusammensetzung nach einem der Ansprüche 1 bis 12 zur Durchführung einer Dauerwellverformung der Haare.
